# EUROPÄISCHE PATENTANMELDUNG

(11) **EP 1 186 657 A1**
(43) Veröffentlichungstag der Anmeldung: **13.03.2002**
(21) Anmeldenummer: 01117811.8
(22) Anmeldetag: 21.07.2001
(51) Int. Cl.: C12N 9/04, C12P 13/08

(54) **Die Nukleotidsequenz des Corynebacterium lldD2-Gens, das für die L-Lactat-Dehydrogenase kodiert**

(30) Priorität: 09.09.2000 DE 10044681
(71) Anmelder: Degussa AG, 40474 Düsseldorf (DE)
(72) Erfinder: Farwick, Mike, Dr., 33615 Bielefeld (DE); Huthmacher, Klaus, Dr., 63571 Gelnhausen (DE); Bathe, Brigitte, Dr., 33154 Salzkotten (DE); Pfefferle, Walter, Dr., 33790 Halle (Westf.) (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
und ein Verfahren zur fermentativen Herstellung von L-Aminosäuren unter Verwendung von coryneformen Bakterien, in denen zumindest das lldD2-Gen verstärkt vorliegt, und die Verwendung von Polynukleotiden, die die erfindungsgemäßen Sequenzen enthalten, als Hybridisierungssonden.

## Beschreibung

Gegenstand der Erfindung sind für das lldD2-Gen kodierende Nukleotidsequenzen aus coryneformen Bakterien und ein Verfahren zur fermentativen Herstellung von Aminosäuren unter Verwendung von Bakterien, in denen das endogene lldD2-Gen verstärkt wird.

### Stand der Technik

L-Aminosäuren, insbesondere L-Lysin, finden in der Humanmedizin und in der pharmazeutischen Industrie, in der Lebensmittelindustrie und ganz besonders in der Tierernährung, Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und Aminosäuren produzieren.

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht.

### Aufgabe der Erfindung

Die Erfinder haben sich zur Aufgabe gestellt, neue Maßnahmen zur verbesserten fermentativen Herstellung von Aminosäuren bereitzustellen.

### Beschreibung der Erfindung

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin gemeint. Besonders bevorzugt ist L-Lysin.

Wenn im folgenden L-Lysin oder Lysin erwähnt werden, sind damit nicht nur die Basen, sondern auch die Salze wie z.B. Lysin-Monohydrochlorid oder Lysin-Sulfat gemeint.

Gegenstand der Erfindung ist ein isoliertes Polynukleotid aus coryneformen Bakterien, enthaltend eine für das lldD2-Gen kodierende Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
wobei das Polypeptid bevorzugt die Aktivität der L-Lactat-Dehydrogenase aufweist.

Gegenstand der Erfindung ist ebenfalls das oben genannte Polynukleotid, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

Weitere Gegenstände sind
ein replizierbares Polynukleotid, insbesondere DNA, enthaltend die Nukleotidsequenz wie in SEQ ID No. 1 dargestellt;
ein Polynukleotid, das für ein Polypeptid kodiert, das die Aminosäuresequenz, wie in SEQ ID No. 2 dargestellt, enthält;
ein Vektor, enthaltend das erfindungsgemäße Polynukleotid, insbesondere Pendelvektor oder Plasmidvektor, und coryneforme Bakterien, die den Vektor enthalten oder in denen das endogene lldD2-Gen verstärkt ist.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank eines coryneformen Bakteriums, die das vollständige Gen oder Teile davon enthält, mit einer Sonde, die die Sequenz des erfindungsgemäßen Polynukleotids gemäß SEQ ID No.1 oder ein Fragment davon enthält und Isolierung der genannten Polynukleotidsequenz.

Polynukleotide, die die Sequenzen gemäß der Erfindung enthalten, sind als Hybridisierungs-Sonden für RNA, cDNA und DNA geeignet, um Nukleinsäuren beziehungsweise Polynukleotide oder Gene in voller Länge zu isolieren, die für die L-Lactat-Dehydrogenase kodieren, oder um solche Nukleinsäuren beziehungsweise Polynukleotide oder Gene zu isolieren, die eine hohe Ähnlichkeit der Sequenz mit der des lldD2-Gens aufweisen.

Polynukleotide, die die Sequenzen gemäß der Erfindung enthalten, sind weiterhin als Primer geeignet, mit deren Hilfe mit der Polymerase-Kettenreaktion (PCR) DNA von Genen hergestellt werden kann, die für die L-Lactat-Dehydrogenase kodieren.

Solche als Sonden oder Primer dienende Oligonukleotide enthalten mindestens 25, 26, 27, 28, 29 oder 30, bevorzugt mindestens 20, 21, 22, 23 oder 24 ganz besonders bevorzugt mindestens 15, 16, 17, 18 oder 19 aufeinanderfolgende Nukleotide. Geeignet sind ebenfalls Oligonukleotide mit einer Länge von mindestens 31, 32, 33, 34, 35, 36, 37, 38, 39 oder 40 oder mindestens 41, 42, 43, 44, 45, 46, 47, 48, 49 oder 50 Nukleotiden. Gegebenenfalls sind auch Oligonukleotide mit einer Länge von mindestens 100, 150, 200, 250 oder 300 Nukleotiden geeignet.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Die Polynukleotide gemäß Erfindung schließen ein Polynukleotid gemäß SEQ ID No. 1 oder ein daraus hergestelltes Fragment und auch solche ein, die zu wenigstens 70% bis 80%, bevorzugt zu wenigstens 81% bis 85%, besonders bevorzugt zu wenigstens 86% bis 90%, und ganz besonders bevorzugt zu wenigstens 91%, 93%, 95%, 97% oder 99% identisch sind mit dem Polynukleotid gemäß SEQ ID No. 1 oder eines daraus hergestellten Fragments.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität der L-Lactat-Dehydrogenase und auch solche ein, die zu wenigstens 70% bis 80%, bevorzugt zu wenigstens 81% bis 85%, besonders bevorzugt zu wenigstens 91%, 93%, 95%, 97% oder 99% identisch mit dem Polypeptid gemäß SEQ ID No. 2 sind und die genannte Aktivität aufweisen.

Die Erfindung betrifft weiterhin ein Verfahren zur fermentativen Herstellung von Aminosäuren, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin, unter Verwendung von coryneformen Bakterien, die insbesondere bereits Aminosäuren produzieren und in denen die für das lldD2-Gen kodierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum (C. glutamicum), sind besonders die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme.

Das neue, für das Enzym L-Lactat-Dehydrogenase (EC 1.1.2.3) kodierende lldD2-Gen von C. glutamicum wurde isoliert.

Zur Isolierung des lldD2-Gens oder auch anderer Gene von C. glutamicum wird zunächst eine Genbank dieses Mikroorganismus in Escherichia coli (E. coli) angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990), oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde.

Börmann et al. (Molecular Microbiology 6(3), 317-326) (1992)) wiederum beschreiben eine Genbank von C. glutamicum ATCC13032 unter Verwendung des Cosmids pHC79 (Hohn und Collins, Gene 11, 291-298 (1980)).

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Die erhaltenen DNA-Sequenzen können dann mit bekannten Algorithmen bzw. Sequenzanalyse-Programmen wie z.B. dem von Staden (Nucleic Acids Research 14, 217-232(1986)), dem von Marck (Nucleic Acids Research 16, 1829-1836 (1988)) oder dem GCG-Programm von Butler (Methods of Biochemical Analysis 39, 74-97 (1998)) untersucht werden.

Die neue für das Gen lldD2 kodierende DNA-Sequenz von C. glutamicum wurde gefunden, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des lldD2-Genproduktes dargestellt.

Kodierende DNA-Sequenzen, die sich aus SEQ ID No. 1 durch die Degeneriertheit des genetischen Kodes ergeben, sind ebenfalls Bestandteil der Erfindung. In gleicher Weise sind DNA-Sequenzen, die mit SEQ ID No. 1 oder Teilen von SEQ ID No. 1 hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" ("sense mutations") bekannt, die zu keiner grundsätzlichen Veränderung der Aktivität des Proteins führen, d.h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID No. 2 ergeben, sind ebenfalls Bestandteil der Erfindung.

In gleicher Weise sind DNA-Sequenzen, die mit SEQ ID No. 1 oder Teilen von SEQ ID No. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Die Hybridisierung findet unter stringenten Bedingungen statt, das heisst, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflußt bzw. bestimmt wird. Die Hybridisierungsreaktion wird vorzugsweise bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C eingestellt wird. Es ist gegebenenfalls möglich die Salzkonzentration bis auf 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90% bis 95% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Anleitungen zur Amplifikation von DNA-Sequenzen mit Hilfe der Polymerase-Kettenreaktion (PCR) findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994).

Es wurde gefunden, daß coryneforme Bakterien nach Überexpression des lldD2-Gens in verbesserter Weise Aminosäuren produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Zur Verstärkung wurde das erfindungsgemäße lldD2-Gen beispielhaft mit Hilfe von episomalen Plasmiden überexprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)), pEM1 (Schrumpf et al, 1991, Journal of Bacteriology 173:4510-4516) oder pBGS8 (Spratt et al.,1986, Gene 41: 337-342) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben.

Nach homologer Rekombination mittels eines "cross over"-Ereignisses enthält der resultierende Stamm mindestens zwei Kopien des betreffenden Gens.

Zusätzlich kann es für die Produktion von L-Aminosäuren vorteilhaft sein, neben dem lldD2-Gen eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine zu verstärken, insbesondere überzuexprimieren.

So kann für die Herstellung von L-Aminosäuren zusätzlich zur Verstärkung des lldD2-Gens eines oder mehrere endogene Gene, ausgewählt aus der Gruppe
- das für die Dihydrodipicolinat-Synthase kodierende Gen dapA (EP-B 0 197 335),
- das für die Glyceraldehyd-3-Phosphat-Dehydrogenase kodierende Gen gap (Eikmanns (1992), Journal of Bacteriology 174:6076-6086),
- das für die Triosephosphat-Isomerase kodierende Gen tpi (Eikmanns (1992), Journal of Bacteriology 174:6076-6086),
- das für die 3-Phosphoglycerat-Kinase kodierende Gen pgk (Eikmanns (1992), Journal of Bacteriology 174:6076-6086),
- das für die Glucose-6-Phosphat-Dehydrogenase kodierende Gen zwf (JP-A-09224661),
- das für die Pyruvat-Carboxylase kodierende Gen pyc (DE-A-198 31 609),
- das für die Malat-Chinon-Oxidoreduktase kodierende Gen mqo (Molenaar et al., European Journal of Biochemistry 254, 395-403 (1998)),
- das für eine feed-back resistente Aspartatkinase kodierende Gen lysC (Accession No.P26512),
- das für den Lysin-Export kodierende Gen lysE (DE-A-195 48 222),
- das für die Homoserin-Dehydrogenase kodierende Gen hom (EP-A 0131171),
- das für die Threonin-Dehydratase kodierende Gen ilvA (Möckel et al., Journal of Bacteriology (1992) 8065-8072)) oder das für eine "feed back resistente" Threonin-Dehydratase kodierende Allel ilvA(Fbr) (Möckel et al., (1994) Molecular Microbiology 13: 833-842),
- das für die Acetohydroxysäure-Synthase kodierenden Gen ilvBN (EP-B 0356739),
- das für die Dihydroxysäuredehydratase kodierende Gen ilvD (Sahm und Eggeling (1999) Applied and Environmental Microbiology 65: 1973-1979),
- das für das Zwal-Protein kodierende Gen zwa1 (DE: 19959328.0, DSM 13115),
verstärkt, insbesondere überexprimiert werden.

Weiterhin kann es für die Produktion von L-Aminosäuren vorteilhaft sein, zusätzlich zur Verstärkung des lldD2-Gens eines oder mehrere Gene, ausgewählt aus der Gruppe
- das für die Phosphoenolpyruvat-Carboxykinase kodierende Gen pck (DE 199 50 409.1; DSM 13047),
- das für die Glucose-6-Phosphat-Isomerase kodierende Gen pgi (US 09/396,478; DSM 12969),
- das für die Pyruvat-Oxidase kodierende Gen poxB (DE: 1995 1975.7; DSM 13114),
- das für das Zwa2-Protein kodierende Gen zwa2 (DE: 19959327.2, DSM 13113)
abzuschwächen, insbesondere die Expression zu verringern.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Massnahmen kombiniert.

Weiterhin kann es für die Produktion von Aminosäuren vorteilhaft sein, neben der Überexpression des lldD2-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Ionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Das erfindungsgemäße Verfahren dient zur fermentativen Herstellung von Aminosäuren.

Die vorliegende Erfindung wird im folgenden anhand von Ausführungsbeispielen näher erläutert.

Die Isolierung von Plasmid-DNA aus Escherichia coli sowie alle Techniken zur Restriktion, Klenow- und alkalische Phosphatasebehandlung wurden nach Sambrook et al. (Molecular Cloning. A Laboratory Manual (1989) Cold Spring Harbour Laboratory Press, Cold Spring Harbor, NY, USA) durchgeführt. Methoden zur Transformation von Escherichia coli sind ebenfalls in diesem Handbuch beschrieben.

Die Zusammensetzung gängiger Nährmedien wie LB- oder TY-Medium kann ebenfalls dem Handbuch von Sambrook et al. entnommen werden.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Diagnostics GmbH, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert.

Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt.

Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 mg/l Ampicillin ausplattiert wurden. Nach Inkubation über Nacht bei 37°C wurden rekombinante Einzelklone selektioniert.

### Beispiel 2

### Isolierung und Sequenzierung des lldD2-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Diagnostics GmbH, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany).

Die DNA des Sequenziervektors pZero-1, bezogen von der Firma Invitrogen (Groningen, Niederlande, Produktbeschreibung Zero Background Cloning Kit, Product No. K2500-01), wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A Laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 mg/l Zeocin ausplattiert.

Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems (Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurde mit dem Programm XNIP (Staden, 1986, Nucleic Acids Research, 14:217-231) angefertigt.

Die erhaltene Nukleotidsequenz ist in SEQ ID No. 1 dargestellt. Die Analyse der Nukleotidsequenz ergab ein offenes Leseraster von 1263 Basenpaaren, welches als lldD2-Gen bezeichnet wurde. Das lldD2-Gen kodiert für ein Protein von 420 Aminosäuren.

SEQUENZPROTOKOLL

<110> Degussa AG

<120> Für das lldD2-Gen kodierende Nukleotidsequenzen

<130> 000501 BT

<140>
<141>

<160> 2

<170> PatentIn Ver. 2.1

<210> 1
<211> 1740
<212> DNA
<213> Corynebacterium glutamicum

<220>
<221> CDS
<222> (259)..(1518)
<223> lldD2-Gen

<400> 1
tcatcgcaga aaacttcgca cctgaggtcc gctacaccgg cgctaccctg ggttaccaag 60

tcggagcagc actcttcggc ggtaccgcac ccattatcgc agcatggctg ttcgaaatct 120

ccggcggaca atggtggcca atcgccgtct acgtcgctgc atgttgcctt ctctctgtga 180

tcgcctcgtt cttcatccaa cgcgtcgcgc accaagagaa ctaaaatcta agtaaaaccc 240

ctccgaaagg aaccaccc atg gtg aaa cgt caa ctg ccc aac ccc gca gaa 291
Met Val Lys Arg Gln Leu Pro Asn Pro Ala Glu
1               5                   10

cta ctc gaa ctc atg aag ttc aaa aag cca gag ctc aac ggc aag aaa 339
Leu Leu Glu Leu Met Lys Phe Lys Lys Pro Glu Leu Asn Gly Lys Lys
            15                  20                  25

cga cgc cta gac tcc gcg ctc acc atc tac gac ctg cgt aaa att gct 387
Arg Arg Leu Asp Ser Ala Leu Thr Ile Tyr Asp Leu Arg Lys Ile Ala
        30                  35                  40

aaa cga cgc acc cca gct gcc gcg ttc gac tac acc gac ggc gca gcc 435
Lys Arg Arg Thr Pro Ala Ala Ala Phe Asp Tyr Thr Asp Gly Ala Ala
    45                  50                  55

gag gcc gaa ctc tca atc aca cgc gca cgt gaa gca ttc gaa aac atc 483
Glu Ala Glu Leu Ser Ile Thr Arg Ala Arg Glu Ala Phe Glu Asn Ile
60                  65                  70                  75

gaa ttc cac cca gac atc ctc aag cct gca gaa cac gta gac acc acc 531
Glu Phe His Pro Asp Ile Leu Lys Pro Ala Glu His Val Asp Thr Thr
                80                  85                  90

acc caa atc ctg ggc gga acc tcc tcc atg cca ttc ggc atc gca cca    579
Thr Gln Ile Leu Gly Gly Thr Ser Ser Met Pro Phe Gly Ile Ala Pro
            95                  100                 105

acc ggc ttc acc cgc ctc atg cag acc gaa ggt gaa atc gca ggt gcc    627
Thr Gly Phe Thr Arg Leu Met Gln Thr Glu Gly Glu Ile Ala Gly Ala
        110                 115                 120

gga gct gca ggc gct gca gga att cct ttc acc ctg tcc acc ctg ggc    675
Gly Ala Ala Gly Ala Ala Gly Ile Pro Phe Thr Leu Ser Thr Leu Gly
    125                 130                 135

act acc tcc atc gaa gac gtc aag gcc acc aac ccc aac ggc cga aac    723
Thr Thr Ser Ile Glu Asp Val Lys Ala Thr Asn Pro Asn Gly Arg Asn
140                 145                 150                 155

tgg ttc cag ctc tac gtc atg cgc gac cgc gaa atc tcc tac ggc ctc    771
Trp Phe Gln Leu Tyr Val Met Arg Asp Arg Glu Ile Ser Tyr Gly Leu
                160                 165                 170

gtc gaa cgc gca gcc aaa gca gga ttc gac acc ctg atg ttc acc gtg    819
Val Glu Arg Ala Ala Lys Ala Gly Phe Asp Thr Leu Met Phe Thr Val
            175                 180                 185

gat acc ccc atc gcc ggc tac cgc atc cgc gat tcc cgc aac gga ttc    867
Asp Thr Pro Ile Ala Gly Tyr Arg Ile Arg Asp Ser Arg Asn Gly Phe
        190                 195                 200

tcc atc ccg cca cag ctg acc cca tcc acc gtg ctc aat gca atc cca    915
Ser Ile Pro Pro Gln Leu Thr Pro Ser Thr Val Leu Asn Ala Ile Pro
    205                 210                 215

cgc cca tgg tgg tgg atc gac ttc ctg acc acc cca acc ctt gag ttc    963
Arg Pro Trp Trp Trp Ile Asp Phe Leu Thr Thr Pro Thr Leu Glu Phe
220                 225                 230                 235

gca tcc ctt tcc tcg acc ggc gga acc gtg ggc gac ctc ctc aac tcc    1011
Ala Ser Leu Ser Ser Thr Gly Gly Thr Val Gly Asp Leu Leu Asn Ser
                240                 245                 250

gcg atg gat ccc acc att tct tac gaa gac ctc aag gtc atc cgt gaa    1059
Ala Met Asp Pro Thr Ile Ser Tyr Glu Asp Leu Lys Val Ile Arg Glu
            255                 260                 265

atg tgg cca ggc aag ctc gta gtc aag ggt gtc cag aac gtt gaa gac    1107
Met Trp Pro Gly Lys Leu Val Val Lys Gly Val Gln Asn Val Glu Asp
        270                 275                 280

tcc gtc aaa ctc ctc gac caa ggc gtc gac ggc ctc atc ctc tcc aac    1155
Ser Val Lys Leu Leu Asp Gln Gly Val Asp Gly Leu Ile Leu Ser Asn
    285                 290                 295

cac ggt ggc cgt caa ctc gac cgc gca cca gtc cca ttc cac ctc ctg    1203
His Gly Gly Arg Gln Leu Asp Arg Ala Pro Val Pro Phe His Leu Leu
300                 305                 310                 315

cca cag gta cgc aag gaa gtc gga tct gaa cca acc atc atg atc gac    1251
Pro Gln Val Arg Lys Glu Val Gly Ser Glu Pro Thr Ile Met Ile Asp
                320                 325                 330

acc ggc atc atg aac ggc gcc gac atc gtc gca gcc gta gcc atg ggc     1299
Thr Gly Ile Met Asn Gly Ala Asp Ile Val Ala Ala Val Ala Met Gly
            335                 340                 345

gct gac ttc acc ctc atc ggt cgt gcc tac ctc tac gga ctc atg gcc     1347
Ala Asp Phe Thr Leu Ile Gly Arg Ala Tyr Leu Tyr Gly Leu Met Ala
        350                 355                 360

gga ggc cgc gaa ggc gtc gac cgc acc atc gcc att ctc cgc agc gag     1395
Gly Gly Arg Glu Gly Val Asp Arg Thr Ile Ala Ile Leu Arg Ser Glu
    365                 370                 375

atc acc cgc acc atg gct ctc ctc ggt gtt tcc tcc ctc gaa gaa ctc     1443
Ile Thr Arg Thr Met Ala Leu Leu Gly Val Ser Ser Leu Glu Glu Leu
380                 385                 390                 395

gag cca cgc cac gtc acc cag ctg gcc aag atg gtt cca gtt tct gac     1491
Glu Pro Arg His Val Thr Gln Leu Ala Lys Met Val Pro Val Ser Asp
                400                 405                 410

gca act cgt tct gca gcg gcg gag att taaaagtttc tctccttagc           1538
Ala Thr Arg Ser Ala Ala Ala Glu Ile
            415                 420

tattaaaagg tgcccatccg tttggatggg caccttctcg tttcttgcaa tcggcatatt 1598

cagtcaaaaa atgttgaaat cagcactttc aatttgggac atctactctt aggagaaaag 1658

ccacaaacct ttcccacccc acaaccgtgt gttctgcagt cgacccagtt tagaggaaac 1718

atgagtgact tcacggaaaa ta                                          1740

<210> 2
<211> 420
<212> PRT
<213> Corynebacterium glutamicum

<400> 2
Met Val Lys Arg Gln Leu Pro Asn Pro Ala Glu Leu Leu Glu Leu Met
1               5                   10                  15

Lys Phe Lys Lys Pro Glu Leu Asn Gly Lys Lys Arg Arg Leu Asp Ser
            20                  25                  30

Ala Leu Thr Ile Tyr Asp Leu Arg Lys Ile Ala Lys Arg Arg Thr Pro
        35                  40                  45

Ala Ala Ala Phe Asp Tyr Thr Asp Gly Ala Ala Glu Ala Glu Leu Ser
    50                  55                  60

Ile Thr Arg Ala Arg Glu Ala Phe Glu Asn Ile Glu Phe His Pro Asp
65                  70                  75                  80

Ile Leu Lys Pro Ala Glu His Val Asp Thr Thr Thr Gln Ile Leu Gly
                85                  90                  95

Gly Thr Ser Ser Met Pro Phe Gly Ile Ala Pro Thr Gly Phe Thr Arg
                100                 105                 110

Leu Met Gln Thr Glu Gly Glu Ile Ala Gly Ala Gly Ala Ala Gly Ala
        115                 120                 125

Ala Gly Ile Pro Phe Thr Leu Ser Thr Leu Gly Thr Thr Ser Ile Glu
    130                 135                 140

Asp Val Lys Ala Thr Asn Pro Asn Gly Arg Asn Trp Phe Gln Leu Tyr
145                 150                 155                 160

Val Met Arg Asp Arg Glu Ile Ser Tyr Gly Leu Val Glu Arg Ala Ala
                165                 170                 175

Lys Ala Gly Phe Asp Thr Leu Met Phe Thr Val Asp Thr Pro Ile Ala
            180                 185                 190

Gly Tyr Arg Ile Arg Asp Ser Arg Asn Gly Phe Ser Ile Pro Pro Gln
        195                 200                 205

Leu Thr Pro Ser Thr Val Leu Asn Ala Ile Pro Arg Pro Trp Trp Trp
    210                 215                 220

Ile Asp Phe Leu Thr Thr Pro Thr Leu Glu Phe Ala Ser Leu Ser Ser
225                 230                 235                 240

Thr Gly Gly Thr Val Gly Asp Leu Leu Asn Ser Ala Met Asp Pro Thr
                245                 250                 255

Ile Ser Tyr Glu Asp Leu Lys Val Ile Arg Glu Met Trp Pro Gly Lys
            260                 265                 270

Leu Val Val Lys Gly Val Gln Asn Val Glu Asp Ser Val Lys Leu Leu
        275                 280                 285

Asp Gln Gly Val Asp Gly Leu Ile Leu Ser Asn His Gly Gly Arg Gln
    290                 295                 300

Leu Asp Arg Ala Pro Val Pro Phe His Leu Leu Pro Gln Val Arg Lys
305                 310                 315                 320

Glu Val Gly Ser Glu Pro Thr Ile Met Ile Asp Thr Gly Ile Met Asn
                325                 330                 335

Gly Ala Asp Ile Val Ala Ala Val Ala Met Gly Ala Asp Phe Thr Leu
            340                 345                 350

Ile Gly Arg Ala Tyr Leu Tyr Gly Leu Met Ala Gly Gly Arg Glu Gly
        355                 360                 365

Val Asp Arg Thr Ile Ala Ile Leu Arg Ser Glu Ile Thr Arg Thr Met
    370                 375                 380

Ala Leu Leu Gly Val Ser Ser Leu Glu Glu Leu Glu Pro Arg His Val
385                 390                 395                 400

Thr Gln Leu Ala Lys Met Val Pro Val Ser Asp Ala Thr Arg Ser Ala
                405                 410                 415

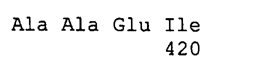

## Patentansprüche

1. Isoliertes Polynukleotid aus coryneformen Bakterien, enthaltend eine für das lldD2-Gen kodierende Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c)
wobei das Polypeptid bevorzugt die Aktivität der L-Lactat-Dehydrogenase aufweist.

2. Polynukleotid gemäß Anspruch 1, wobei das Polynukleotid eine in coryneformen Bakterien replizierbare, bevorzugt rekombinante DNA ist.

3. Polynukleotid gemäß Anspruch 1, wobei das Polynukleotid eine RNA ist.

4. Polynukleotid gemäß Anspruch 2, enthaltend die Nukleinsäuresequenz wie in SEQ ID No. 1 dargestellt.

5. Replizierbare DNA gemäß Anspruch 2, enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

6. Replizierbare DNA gemäß Anspruch 5, **dadurch gekennzeichnet, daß** die Hybridisierung unter einer Stringenz entsprechend höchstens 2x SSC durchgeführt wird.

7. Polynukleotidsequenz gemäß Anspruch 1, die für ein Polypeptid kodiert, das die in SEQ ID No. 2 dargestellte Aminosäuresequenz enthält.

8. Coryneforme Bakterien, in denen das lldD2-Gen verstärkt, insbesondere überexprimiert wird.

9. Verfahren zur fermentativen Herstellung von L-Aminosäuren, insbesondere L-Lysin, **dadurch gekennzeichnet, daß** man folgende Schritte durchführt:
a) Fermentation der die gewünschte L-Aminosäure produzierenden coryneformen Bakterien, in denen man zumindest das endogene lldD2-Gen oder dafür kodierende Nukleotidsequenzen verstärkt, insbesondere überexprimiert;
b) Anreicherung der L-Aminosäure im Medium oder in den Zellen der Bakterien, und
c) Isolierung der L-Aminosäure.

10. Verfahren gemäß Anspruch 9, **dadurch gekennzeichnet, daß** man Bakterien einsetzt, in denen man zusätzlich weitere Gene des Biosyntheseweges der gewünschten L-Aminosäure verstärkt.

11. Verfahren gemäß Anspruch 9, **dadurch gekennzeichnet, daß** man Bakterien einsetzt, in denen die Stoffwechselwege zumindest teilweise ausgeschaltet sind, die die Bildung der gewünschten L-Aminosäure verringern.

12. Verfahren gemäß Anspruch 9, **dadurch gekennzeichnet, daß** man einen mit einem Plasmidvektor transformierten Stamm einsetzt, und der Plasmidvektor die für das lldD2-Gen kodierende Nukleotidsequenz trägt.

13. Verfahren gemäß Anspruch 9, **dadurch gekennzeichnet, daß** man die Expression des (der) Polynukleotides (e), das (die) für das lldD2-Gen kodiert (kodieren) verstärkt, insbesondere überexprimiert.

14. Verfahren gemäß Anspruch 9, **dadurch gekennzeichnet, daß** man die katalytischen Eigenschaften des Polypetids (Enzymprotein) erhöht, für das das Polynukleotid lldD2 kodiert.

15. Verfahren gemäß Anspruch 9, **dadurch gekennzeichnet, daß** man zur Herstellung von L-Aminosäuren coryneforme Mikroorganismen fermentiert, in denen man gleichzeitig eines oder mehrere der endogenen Gene, ausgewählt aus der Gruppe
15.1 das für die Dihydrodipicolinat-Synthase kodierende Gen dapA,
15.2 das für die Glyceraldehyd-3-Phosphat-Dehydrogenase kodierende Gen gap,
15.3 das für die Triosephosphat-Isomerase kodierende Gen tpi,
15.4 das für die 3-Phosphoglycerat-Kinase kodierende Gen pgk,
15.5 das für die Glucose-6-Phosphat-Dehydrogenase kodierende Gen zwf,
15.6 das für die Pyruvat-Carboxylase kodierende Gen pyc,
15.7 das für die Malat-Chinon-Oxidoreduktase kodierende Gen mqo,
15.8 das für eine feed-back resistente Aspartatkinase kodierende Gen lysC,
15.9 das für den Lysin-Export kodierende Gen lysE,
15.10 das für die Homoserin-Dehydrogenase kodierende Gen hom,
15.11 das für die Threonin-Dehydratase kodierende Gen ilvA oder das für eine feed back resistente Threonin-Dehydratase kodierende Allel ilvA(Fbr),
15.12 das für die Acetohydroxysäure-Synthase kodierende Gen ilvBN,
15.13 das für die Dihydroxysäuredehydratase kodierende Gen ilvD,
15.14 das für das Zwal-Protein kodierende Gen zwa1 verstärkt bzw. überexprimiert.

16. Verfahren gemäß Anspruch 9, **dadurch gekennzeichnet, daß** man zur Herstellung von L-Aminosäuren coryneforme Mikroorganismen fermentiert, in denen man gleichzeitig eines oder mehrere der Gene, ausgewählt aus der Gruppe
16.1 das für die Phosphoenolpyruvat-Carboxykinase kodierende Gen pck,
16.2 das für die Glucose-6-Phosphat Isomerase kodierende Gen pgi,
16.3 das für die Pyruvat-Oxidase kodierende Gen poxB
16.4 das für das Zwa2-Protein kodierende Gen zwa2 abschwächt.

17. Coryneforme Bakterien, die einen Vektor enthalten, der ein Polynukleotid gemäß Anspruch 1 trägt.

18. Verfahren gemäß einem oder mehreren der Ansprüche 9-16, **dadurch gekennzeichnet, daß** man Mikroorganismen der Art Corynebacterium glutamicum einsetzt.

19. Verfahren zum Auffinden von RNA, cDNA und DNA, um Nukleinsäuren, beziehungsweise Polynukleotide oder Gene zu isolieren, die für die L-Lactat-Dehydrogenase kodieren oder eine hohe Ähnlichkeit mit der Sequenz des lldD2-Gens aufweisen, **dadurch gekennzeichnet,** daß man das Polynukleotid, enthaltend die Polynukleotidsequenzen gemäß den Ansprüchen 1, 2, 3 oder 4, als Hybridisierungssonden einsetzt.

20. Verfahren gemäß Anspruch 19, **dadurch gekennzeichnet, daß** man arrays, micro arrays oder DNA-chips einsetzt.
